(19) Europäisches Patentamt
European Patent Office
Office européen des brevets

(11) EP 0 670 302 B1

(12) EUROPÄISCHE PATENTSCHRIFT

(45) Veröffentlichungstag und Bekanntmachung des Hinweises auf die Patenterteilung:
**11.02.1998 Patentblatt 1998/07**

(51) Int Cl.[6]: **C07C 233/18**

(21) Anmeldenummer: **95102324.1**

(22) Anmeldetag: **20.02.1995**

(54) **N-Acyloxyalkyl-carbonsäureamide und Verfahren zu ihrer Herstellung**

N-acyloxylalkyl carboxylic acid amides and process for their preparation

Amides-N-acyloxy alkyliques d'acides carboxyliques et procédé de leur préparation

(84) Benannte Vertragsstaaten:
**AT BE CH DE DK ES FR GB IT LI NL**

(30) Priorität: **04.03.1994 DE 4407182**

(43) Veröffentlichungstag der Anmeldung:
**06.09.1995 Patentblatt 1995/36**

(73) Patentinhaber: **BAYER AG**
**51368 Leverkusen (DE)**

(72) Erfinder:
- **Lantzsch, Reinhard, Dr.**
  **D-42115 Wuppertal (DE)**
- **Lindner, Werner, Dr.**
  **D-51067 Köln (DE)**

(56) Entgegenhaltungen:
**CH-A- 428 700**
**US-A- 2 980 688**
**US-A- 3 317 603**

- **RECUEIL DES TRAVAUX CHIMIQUES DES PAYS-BAS, Bd.79, 1960, DEN HAAG NL Seiten 401 - 407 H. BREEDERVELD 'The chemistry of the N-alkylaldimines. I. The reaction of N-alkylaldimines with acetic anhydride'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 670 302 B1

## Beschreibung

Die Erfindung betrifft neue N-Acyloxyalkyl-carbonsäureamide, welche als Zwischenprodukte zur Herstellung von agrochemischen oder pharmazeutischen Wirkstoffen verwendet werden können, und ein Verfahren zu ihrer Herstellung.

Es ist aus der Literatur bekannt, daß N-Acyloxyalkyl-carbonsäureamide wegen ihrer mangelnden Stabilität nicht isoliert werden können (vgl. Recueil Trav. Chim. Pays Bas 79, 402; US-P 3317603).

Gegenstand der vorliegenden Erfindung sind

(1) neue N-Acyloxyalkyl-carbonsäureamide der allgemeinen Formel (I)

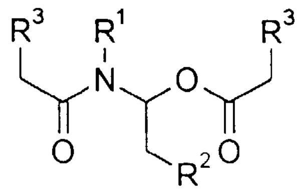

(I)

in welcher

$R^1$ für jeweils gegebenenfalls substituiertes Alkyl, Alkenyl, Alkinyl, Cycloalkyl, Cycloalkylalkyl, Arylalkyl oder Heteroarylalkyl steht,

$R^2$ für gegebenenfalls substituiertes Alkyl steht und

$R^3$ für Wasserstoff, Halogen oder gegebenenfalls substituiertes Alkyl steht,

in isolierter und reiner (mindestens 90 Gew.-%) Form,

(2) ein Verfahren zur Herstellung von N-Acyloxyalkyl-carbonsäureamiden der allgemeinen Formel (I)

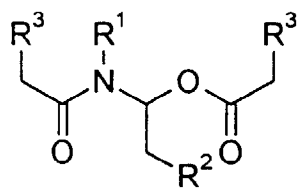

(I)

in welcher

$R^1$ für jeweils gegebenenfalls substituiertes Alkyl, Alkenyl, Alkinyl, Cycloalkyl, Cycloalkylalkyl, Arylalkyl oder Heteroarylalkyl steht,

$R^2$ für gegebenenfalls substituiertes Alkyl steht, und

$R^3$ für Wasserstoff, Halogen oder gegebenenfalls substituiertes Alkyl steht,

dadurch gekennzeichnet, daß man Imine der allgemeinen Formel (II)

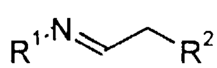

(II)

in welcher

$R^1$ und $R^2$ die oben angegebene Bedeutung haben,

mit Anhydriden der allgemeinen Formel (III)

$$(R^3\text{-}CH_2\text{-}CO)_2O \qquad (III)$$

in welcher

$R^3$ die oben angegebene Bedeutung hat,

in Gegenwart von 0,3 bis 0,9 Mol pro Mol Imin der Formel (II) einer Base und gegebenenfalls in Gegenwart eines Verdünnungsmittels bei Temperaturen zwischen -30°C und +50°C umsetzt.

In Anbetracht des oben zitierten Standes der Technik (Recueil Trav. Chim. Pays Bas 79, 402) ist es als ausgesprochen überraschend anzusehen, daß nach dem erfindungsgemäßen Verfahren die N-Acyloxyalkyl-carbonsäureamide der Formel (I) als isolierbare Produkte in hohen Ausbeuten und in guter Qualität erhalten werden können. Im Gegenstand zum Stand der Technik, gemäß welchem man die Base äquimolar oder im Überschuß verwendet, wird beim erfindungsgemäßen Verfahren die Base im Unterschuß eingesetzt.
Die Umwandlung zu entsprechenden Enamiden (unten), welche als Zwischenprodukte für Agrochemikalien und Arzneimittel-Wirkstoffe verwendet werden können, gelingt mit wesentlich besseren Ausbeuten als nach bekannten Methoden (vgl. J. Chem. Soc. Perkin Trans. I 1984, 1173-1182). Das erfindungsgemäße Verfahren stellt somit eine wesentliche Bereicherung des Standes der Technik dar.

Die Erfindung betrifft vorzugsweise nach dem erfindungsgemäßen Verfahren herzustellende neue N-Acyloxyalkyl-carbonsäureamide der Formel (I), in welcher

$R^1$ für gegebenenfalls durch Halogen oder $C_1$-$C_4$-Alkoxy substituiertes Alkyl mit 1 bis 6 Kohlenstoffatomen, für jeweils gegebenenfalls durch Halogen substituiertes Alkenyl oder Alkinyl mit jeweils 3 bis 6 Kohlenstoffatomen, für jeweils gegebenenfalls durch Halogen oder $C_1$-$C_4$-Alkyl substituiertes Cycloalkyl oder Cycloalkylalkyl mit jeweils 3 bis 6 Kohlenstoffatomen in den Cycloalkylteilen und gegebenenfalls 1 bis 4 Kohlenstoffatomen im Alkylteil, oder für jeweils gegebenenfalls durch Halogen, $C_1$-$C_4$-Alkyl oder $C_1$-$C_4$-Alkoxy substituiertes Arylalkyl mit 6 oder 10 Kohlenstoffatomen im Arylteil und 1- bis 4 Kohlenstoffatomen im Alkylteil oder Heteroarylalkyl mit 3 bis 5 Kohlenstoffatomen und 1 bis 3 Heteroatomen - insbesondere Stickstoff, Sauerstoff oder Schwefel - im Heteroarylteil und 1 bis 4 Kohlenstoffatomen im Alkylteil steht,

$R^2$ für gegebenenfalls durch Halogen oder $C_1$-$C_4$-Alkoxy substituiertes Alkyl mit 1 bis 6 Kohlenstoffatomen steht, und

$R^3$ für Wasserstoff, Halogen oder gegebenenfalls durch Halogen oder $C_1$-$C_4$-Alkoxy substituiertes Alkyl mit 1 bis 4 Kohlenstoffatomen steht.

Die Erfindung betrifft insbesondere nach dem erfindungsgemäßen Verfahren herzustellende neue N-Acyloxyalkyl-carbonsäureamide der Formel (I), in welcher

$R^1$ für jeweils gegebenenfalls durch Fluor, Chlor, Methoxy oder Ethoxy substituiertes Methyl, Ethyl, n- oder i-Propyl, n-, i- oder s-Butyl, für jeweils gegebenenfalls durch Fluor, Chlor oder Brom substituiertes Propenyl, Butenyl, Propinyl oder Butinyl, für jeweils gegebenenfalls durch Fluor, Chlor, Brom, Methyl, Ethyl, n- oder i-Propyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Fluor, Chlor, Brom, Methyl, Ethyl, n- oder i-Propyl, n-, i-, s- oder t-Butyl, Methoxy oder Ethoxy substituiertes Benzyl, Phenylethyl, Phenylpropyl, Naphthylmethyl, Thienylmethyl oder Pyridylmethyl steht,

$R^2$ für jeweils gegebenenfalls durch Fluor, Chlor, Methoxy oder Ethoxy substituiertes Methyl, Ethyl, n- oder i-Propyl, n-, i-, s- oder t-Butyl steht, und

$R^3$ für Wasserstoff, Fluor, Chlor, Brom, oder für jeweils gegebenenfalls durch Fluor, Chlor, Methoxy oder Ethoxy

substituiertes Methyl, Ethyl, n- oder i-Propyl, n-, i-, s- oder t-Butyl steht.

Verwendet man beispielsweise N-Methylpropanaldimin und Acetanhydrid als Ausgangsstoffe, so kann der Reaktionsablauf bei erfindungsgemäßen Verfahren durch das folgende Formelschema skizziert werden:

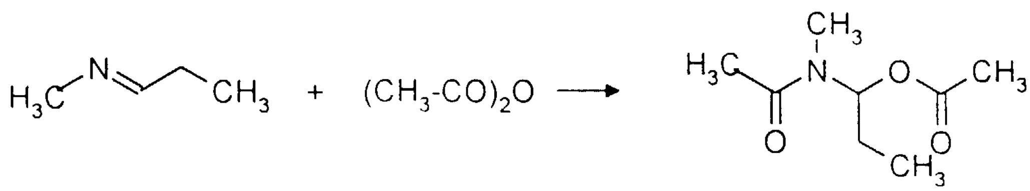

Die beim erfindungsgemäßen Verfahren zur Herstellung der Verbindungen der allgemeinen Formel (I) als Ausgangsstoffe zu verwendenden Imine sind durch die Formel (II) allgemein definiert. In der Formel (II) haben $R^1$ und $R^2$ vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für $R^1$ und $R^2$ angegeben wurden.

Die Ausgangsstoffe der Formel (II) sind bekannt und/oder können nach an sich bekannten Verfahren hergestellt werden (vgl. EP-A 546418).

Die beim erfindungsgemäßen Verfahren weiter als Ausgangsstoffe zu verwendenden Anhydride sind durch die Formel (III) allgemein definiert. In der Formel (II) hat $R^3$ vorzugsweise bzw. insbesondere diejenige Bedeutung, die bereits oben im Zusammenhang mit der Beschreibung der Verbindungen der Formel (I) als bevorzugt bzw. als insbesondere bevorzugt für $R^3$ angegeben wurde.

Die Ausgangsstoffe der Formel (III) sind bekannte organische Synthesechemikalien.

Das erfindungsgemäße Verfahren wird vorzugsweise in Gegenwart einer Base durchgeführt. Es kommen hierbei insbesondere basische organische Stickstoffverbindungen in Betracht. Hierzu gehören beispielsweise Trimethylamin, Triethylamin, Tripropylamin, Tributylamin, Dimethylbutylamin, Dibutylmethylamin, N,N-Dimethyl-anilin, N,N-Dimethylbenzylamin, Pyridin, Picoline, Dimethylpyridine, N-Methyl-piperidin, N-Methyl-morpholin, N,N-Dimethylamino-pyridin und N,N-Dimethyl-cyclohexylamin.

Das erfindungsgemäße Verfahren wird vorzugsweise in Gegenwart eines Verdünnungsmittels durchgeführt. Es kommen hierbei insbesondere mit Wasser praktisch nicht mischbare organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Heptan, Cyclohexan, Methylcyclohexan, Dichlormethan, Chloroform und Tetrachlormethan; Ether, wie beispielsweise Diethylether, Diisopropylether, t-Butyl-methylether, t-Amyl-methylether, Dioxan, Tetrahydrofuran und Ethylenglykoldimethyl- oder -diethylether; Ketone, wie beispielsweise Methyl-isopropyl-keton und Methyl-isobutyl-keton; sowie Ester wie beispielsweise Essigsäure-methylester, -ethylester, -n- oder i-propylester, -n-, -i- und -s-butylester.

Die Reaktionstemperaturen können bei der Durchführung des erfindungsgemäßen Verfahrens in einem größeren Bereich variiert werden. Im allgemeinen arbeitet man bei Temperaturen zwischen -30°C und +50°C, vorzugsweise bei Temperaturen zwischen 0°C und +30°C, insbesondere bei Temperaturen zwischen 10°C und 20°C.

Das erfindungsgemäße Verfahren wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens zur Herstellung der Verbindungen der Formel (I) setzt man pro Mol an Imin der Formel (II) im allgemeinen 0,8 bis 1,2 Mol, vorzugsweise 1,00 bis 1,10 Mol an Anhydrid der Formel (III) und 0,3 bis 0,9 Mol einer Base ein.

In einer bevorzugten Ausführungsform des erfindungsgemäßen Verfahrens wird das Anhydrid der Formel (III) in einem geeigneten Verdünnungsmittel vorgelegt, die Lösung eines Imins der Formel (II) und einer Base im gleichen Verdünnungsmittel wird im unteren Teil des oben angegebenen Temperaturbereichs eindosiert und das Reaktionsgemisch bis zum Ende der Umsetzung gerührt.

Dann werden die leichter flüchtigen Komponenten - Lösungsmittel und gegebenenfalls Base - unter vermindertem Druck abdestilliert, wobei die Temperatur 50°C nicht übersteigen sollte. Der verbleibende Rückstand enthält dann das Produkt der Formel (I).

Die nach dem erfindungsgemäßen Verfahren herzustellenden N-Acyloxyalkyl-carbonsäureamide der Formel (I) können durch Pyrolyse, d.h. durch Erhitzen auf Temperaturen zwischen 100°C und 200°C, vorzugsweise zwischen 120°C und 150°C, unter vermindertem Druck - im allgemeinen zwischen 0,1 mbar und 100 mbar, vorzugsweise zwischen 1 mbar und 30 mbar - in entsprechende Enamide der allgemeinen Formel (IV)

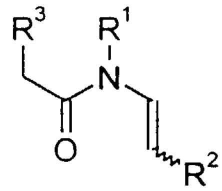

(IV)

in welcher

R[1], R[2] und R[3] die oben angegebene Bedeutung haben,

umgewandelt werden (vgl. die Herstellungsbeispiele), welche als Vorprodukte für Insektizide bekannt sind (vgl. EP-A 546418).

**Herstellungsbeispiele:**

**Beispiel 1**

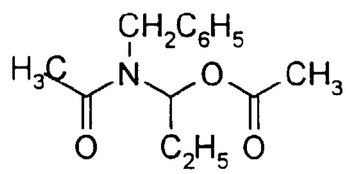

58,9 g (0,4 Mol) N-Benzyl-propanaldimin werden in 100 ml Toluol gelöst und mit 25,4 g (0,2 Mol) N,N-Dimethyl-cyclohexylamin versetzt. Diese Lösung wird bei 10-15°C zu 40,8 g (0,4 Mol) Acetanhydrid getropft. Man läßt auf Raumtemperatur kommen und destilliert Toluol und N,N-Dimethyl-cyclohexylamin im Vakuum (zunächst 12 mbar, dann 1-2 mbar) bei maximal 50°C ab.

Man erhält als Rückstand 99,4 g eines klaren Öls, das aus N-Benzyl-N-(1-acetoxypropyl)-acetamid besteht. Dies entspricht einer Ausbeute von 99% der Theorie.
$^1$H-NMR ($CDCl_3$, d): 0,9 ppm (t, $CH_3$), 1,65 ppm (s, $CH_3$), 1,8 ppm (m, $CH_2$), 2,4 ppm (s, $CH_3$), 4,35 und 4,85 (dd, $CH_2$), 6,25 ppm (t, CH), 7,1-7,4 ppm (m, $C_6H_5$).

**Beispiel 2**

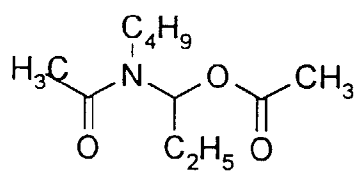

45,28 g (0,4 Mol) N-Butyl-propanaldimin werden in 100 ml Toluol gelöst und mit 25,25 g (0,25 Mol) Triethylamin versetzt. Diese Lösung wird bei 10-15°C zu 40,8 g (0,4 Mol) Acetanhydrid getropft. Man läßt auf Raumtemperatur kommen und destilliert Toluol und Triethylamin zuerst im Wasserstrahlvakuum, dann im Hochvakuum (1-2 mbar) bei maximal 40°C ab.

Man erhält 69 g N-Butyl-N(1-acetoxypropyl)-acetamid als farbloses klares Öl.
$n_D^{20}$ = 1,445

Durch Erhitzen analog Beispiel 2 erhält man N-Butyl-N-propenyl-acetamid in einer Ausbeute von 94% der Theorie (bezogen auf Aldimin).

Analog zu den Beispielen 1 und 2 sowie entsprechend der allgemeinen Beschreibung des erfindungsgemäßen Verfahrens können beispielsweise auch die in der nachstehenden Tabelle 1 aufgeführten Verbindungen der Formel

(I) hergestellt werden.

**Tabelle 1:** Beispiele für die Verbindungen der Formel (I)

| Bsp.-Nr. | Strukturformel | Brechungsindex |
|---|---|---|
| 3 | 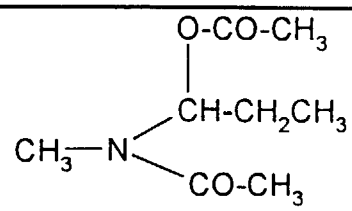 | 1,441 |
| 4 | 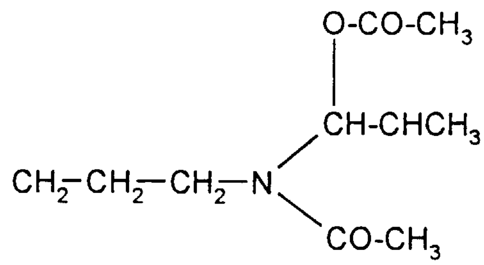 | 1,458 |
| 5 | 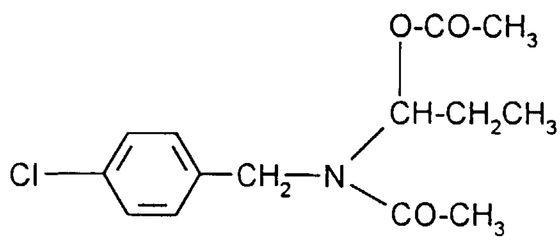 | 1,530 |
| 6 | 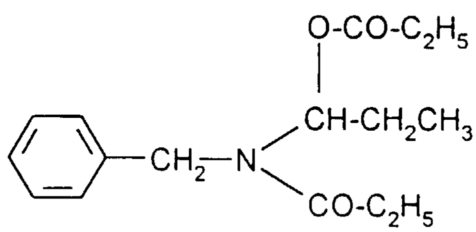 | 1,512 |
| 7 | 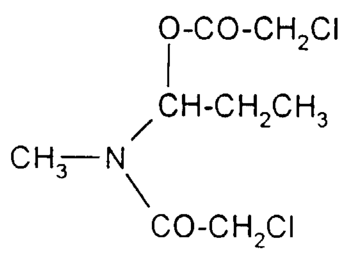 | 1,498 |
| 8 | 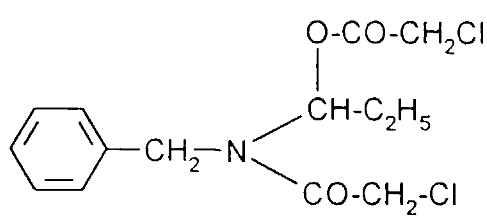 | 1,516 |

**Enamide der Formel (IV):**

**Beispiel (IV-1)**

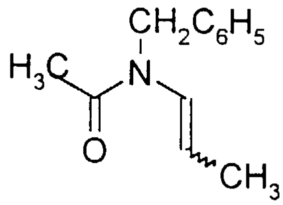

74,8 g des Produkts aus Beispiel 1 werden in einer Destillationsapparatur unter vermindertem Druck (ca. 5 mbar) 5 Stunden auf 130°C erhitzt. Die sich dabei bildende Essigsäure destilliert ab. Das zurückbleibende N-Benzyl-N-propenylacetamid wiegt 56,7 g und hat einen Gehalt von 97% (HPLC).

Analog Beispiel (IV-1) können beispielsweise auch die in der nachstehenden Tabelle 2 aufgeführten Verbindungen der Formel (IV) hergestellt werden.

**Tabelle 2:** Beispiele für die Verbindungen der Formel (IV)

| Bsp.-Nr. | Strukturformel | Brechungsindex |
|---|---|---|
| IV-2 | 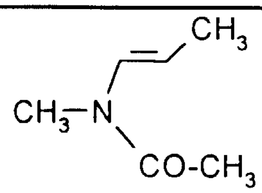 | 1,4794 |
| IV-3 | 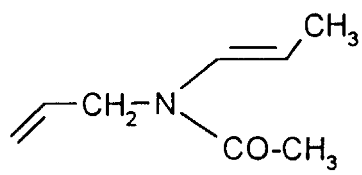 | 1,4846 |
| IV-4 | 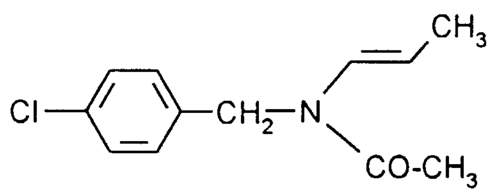 | |
| IV-5 | 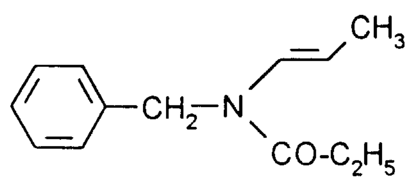 | |
| IV-6 | 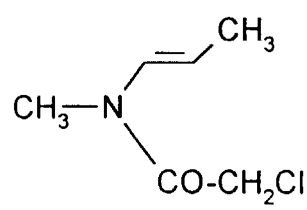 | |
| IV-7 | 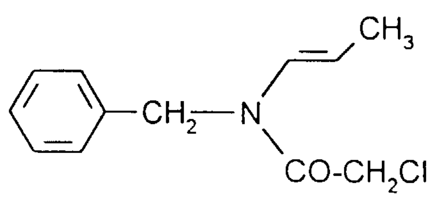 | |
| IV-8 | 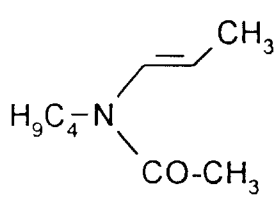 | 1,4640 |

## Patentansprüche

1. Isolierte, reine N-Acyloxyalkyl-carbonsäureamide der allgemeinen Formel (I)

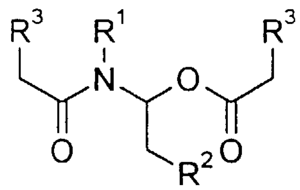

(I)

in welcher

$R^1$ für jeweils gegebenenfalls substituiertes Alkyl, Alkenyl, Alkinyl, Cycloalkyl, Cycloalkylalkyl, Arylalkyl oder Heteroarylalkyl steht,

$R^2$ für gegebenenfalls substituiertes Alkyl steht und

$R^3$ für Wasserstoff, Halogen oder gegebenenfalls substituiertes Alkyl steht.

2. Verfahren zur Herstellung von N-Acyloxyalkyl-carbonsäureamiden der allgemeinen Formel (I)

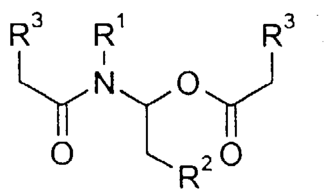

(I)

in welcher

$R^1$ für jeweils gegebenenfalls substituiertes Alkyl, Alkenyl, Alkinyl, Cycloalkyl, Cycloalkylalkyl, Arylalkyl oder Heteroarylalkyl steht,

$R^2$ für gegebenenfalls substituiertes Alkyl steht, und

$R^3$ für Wasserstoff, Halogen oder gegebenenfalls substituiertes Alkyl steht,

dadurch gekennzeichnet, daß man Imine der allgemeinen Formel (II)

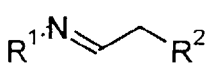

(II)

in welcher

$R^1$ und $R^2$ die oben angegebene Bedeutung haben,

mit Anhydriden der allgemeinen Formel (III)

$$(R^3\text{-}CH_2\text{-}CO)_2O \qquad (III)$$

in welcher

$R^3$ die oben angegebene Bedeutung hat,

in Gegenwart von 0,3 bis 0,9 Mol pro Mol Imin der Formel (II) einer Base und gegebenenfalls in Gegenwart eines Verdünnungsmittels bei Temperaturen zwischen -30°C und +50°C umsetzt, und die Verbindungen der Formel (I) isoliert.

## Claims

**1.** Isolated pure N-acyloxyalkyl-carboxamides of the general formula (I):

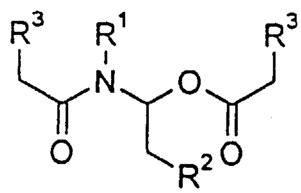

(I)

in which

$R^1$ is alkyl, alkenyl, alkinyl, cycloalkyl, cycloalkylalkyl, arylalkyl or heteroarylalkyl, each of which is optionally substituted,

$R^2$ is optionally substituted alkyl and

$R^3$ is hydrogen, halogen or optionally substituted alkyl.

**2.** Process for the preparation of N-acyloxyalkyl-carboxamides of the general formula (I):

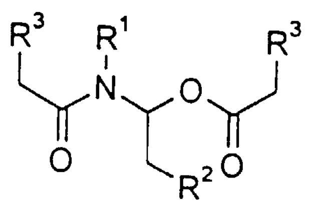

(I)

in which

$R^1$ is alkyl, alkenyl, alkinyl, cycloalkyl, cycloalkylalkyl, arylalkyl or heteroarylalkyl, each or which is optionally substituted,

$R^2$ is optionally substituted alkyl and

$R^3$ is hydrogen, halogen or optionally substituted alkyl,

characterized in that imines of the general formula (II) :

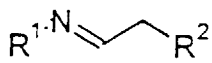

(II)

in which

$R^1$ and $R^2$ are as defined above,

are reacted with anhydrides of the general formula (III):

$$(R^3\text{-}CH_2\text{-}CO)_2O \qquad \text{(III)}$$

in which

$R^3$ is as defined above,

in the presence of 0.3 to 0.9 mol per mole of imine of the formula II of a base and optionally in the presence of a diluent, at temperatures between -30°C and +50°C, and the compounds of the formula (I) are isolated.

**Revendications**

1. Amides d'acides N-acyloxyalkylcarboxyliques purs, isolés répondant à la formule générale (I)

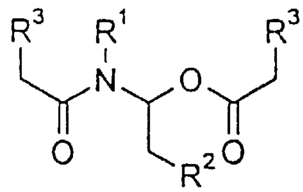

(I)

dans laquelle

$R^1$ représente un groupe alkyle, un groupe alcényle, un groupe alcynyle, un groupe cycloalkyle, un groupe cycloalkylalkyle, un groupe arylalkyle ou un groupe hétéroarylalkyle, chacun de ces groupes portant éventuellement un ou plusieurs substituants identiques ou différents,

$R^2$ représente un groupe alkyle portant éventuellement un ou plusieurs substituants identiques ou différents, et

$R^3$ représente un atome d'hydrogène, un atome d'halogène ou encore un groupe alkyle portant éventuellement un ou plusieurs substituants identiques ou différents.

2. Procédé pour la préparation d'amides d'acides N-acyloxyalkylcarboxyliques répondant à la formule générale (I)

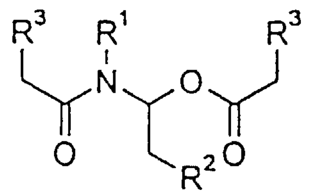

(I)

dans laquelle

$R^1$ représente un groupe alkyle, un groupe alcényle, un groupe alcynyle, un groupe cycloalkyle, un groupe cycloalkylalkyle, un groupe arylalkyle ou un groupe hétéroarylalkyle, chacun de ces groupes portant éventuellement un ou plusieurs substituants identiques ou différents,

$R^2$ représente un groupe alkyle portant éventuellement un ou plusieurs substituants identiques ou différents, et

$R^3$ représente un atome d'hydrogène, un atome d'halogène ou encore un groupe alkyle portant éventuellement un ou plusieurs substituants identiques ou différents,

caractérisé en ce qu'on fait réagir des imines répondant à la formule générale (II)

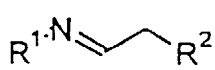

(II)

dans laquelle

$R^1$ et $R^2$ ont la signification indiquée ci-dessus,

avec des anhydrides répondant à la formule générale (III)

$$(R^3\text{-}CH_2\text{-}CO)_2O \qquad \text{(III)}$$

dans laquelle

$R^3$ a la signification indiquée ci-dessus,

en présence de 0,3 à 0,9 mole par mole d'imine de formule (II) d'une base et, le cas échéant, en présence d'un diluant, à des températures entre -30°C et +50°C, et on isole les composés de formule (I).